(19) Europäisches Patentamt
European Patent Office
Office européen des brevets

(11) Publication number : **0 604 181 A1**

# (12) EUROPEAN PATENT APPLICATION

(21) Application number : **93310354.1**

(22) Date of filing : **20.12.93**

(51) Int. Cl.$^5$ : **C07D 471/06,** A61K 31/47, // (C07D471/06, 235:00, 221:00)

(30) Priority : **21.12.92 US 994397**

(43) Date of publication of application : **29.06.94 Bulletin 94/26**

(84) Designated Contracting States : **AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(72) Inventor : **Campbell, Jack Beuford**
**1809 South Chester**
**Indianapolis, Indiana 46203 (US)**
Inventor : **Grindey, Gerald Burr**
**deceased (US)**
Inventor : **Lavagnino, Edward Ralph**
**4010 Rommel Drive**
**Indianapolis, Indiana 46208 (US)**
Inventor : **Merriman, Ronald Lynn**
**5246 Wilton Wood Court**
**Indianapolis, Indiana 46254 (US)**
Inventor : **Poore, Gerald Auston**
**1065 Old Moore Road**
**Martinsvilee, Indiana 46151 (US)**
Inventor : **Shih, Chuan**
**12532 Pebble Pointe Pass**
**Carmel, Indiana 46033 (US)**
Inventor : **Swift, Robert Alan**
**11229 Ashley Lane**
**Fishers, Indiana 46038 (US)**

(74) Representative : **Tapping, Kenneth George et al**
**Erl Wood Manor**
**Windlesham Surrey, GU20 6PH (GB)**

## (54) Antitumor compositions and method of treatment.

(57) This invention provides a series of novel substituted 7H-benzimidazo[2,1-*a*]benz[*de*]isoquinoline-7-ones, and the pharmaceutically acceptable salts or solvates thereof. This invention further provides methods of treating susceptible neoplasms in mammals using these 7H-benzimidazo[2,1-*a*]benz[*de*]isoquinoline-7-ones. This invention additionally provides novel pharmaceutical formulations comprising one or more of the compounds of this invention in combination with pharmaceutically acceptable diluents, excipients and carriers.

EP 0 604 181 A1

In recent years fundamental advances have been made in the development of chemical agents and regimens of therapy to combat neoplastic diseases. Despite these continuing advances, cancers continue to exact intolerable levels of human pain and suffering. The need for new and better methods of treating neoplasms and leukemias continues to fuel efforts to find new classes of antitumor compounds, especially in the areas of inoperable and metastatic solid tumors, such as the various forms of lung cancer. Of the one million new cases of cancer diagnosed in the United States each year, more than 90% represent non-hematopoetic tumors, where improvements in five-year survival rates have been modest, at best. B.E. Henderson, et al., Science, 254:1131-1137 (1991).

The recent avalanche of information regarding the basic biological processes involved in neoplasms has led to a deeper understanding of the heterogeneity of tumors. Ongoing work has led to the realization that individual tumors may contain many subpopulations of neoplastic cells that differ in crucial characteristics such as karyotype, morphology, immunogenicity, growth rate, capacity to metastasize, and response to antineoplastic agents.

It is because of this extreme heterogeneity among populations of neoplastic cells that new chemotherapeutic agents should have a wide spectrum of activity and a large therapeutic index. In addition, such agents must be chemically stable and compatible with other agents. It is also important that any chemotherapeutic regimen be as convenient and painless as possible to the patient.

Since 1949 approximately 50 drugs have been introduced in the United States for the treatment of cancers. These drugs have increased the survival of some cancer patients, particularly those with leukemias or testicular carcinoma. These drugs, however, are not generally effective against commonly occurring carcinomas, especially lung, colon, and breast cancers. Also, the available cancer drugs have a high toxicity due to their lack of cancer cell selectivity.

Several drug discovery strategies have been proposed to overcome the shortfalls of the current anticancer drugs. One strategy is to screen for drugs that are more effective against the commonly occurring carcinomas. Another is to screen for drugs that regulate, or kill, tumor cells but not normal cells. For these drug development strategies to work, the pharmacological target must have several attributes. First, the target should be qualitatively different in neoplastic cells than normal cells. Second, the target must be essential for the maintenance of the cancer phenotype. Recent advances in the molecular biology of cancer now make it possible to identify pharmacological targets with these attributes.

Over the past decade there have been significant advances in the enzymatic and chemical techniques used in molecular biology. With these techniques, investigators are now able to look at the genome of human cells and identify specific molecular differences between normal and transformed cells.

Proto-oncogenes are genes that have been found to differ in normal and transformed cells. These are a discrete set of normal genes which are activated into transforming oncogenes, and are thought to cause a wide variety of human cancers. A variety of oncogene products have been identified. These gene products include growth factors, growth factor receptors, protein kinases and guanosine triphosphate binding proteins. All of these gene products are potential targets for new anticancer drugs.

The proto-oncogenes can be classified either by the function and the cellular location of their encoded proteins, or by the mechanisms that cause their activation to transforming oncogenes. For the discovery of anticancer drugs with high tumor selectivity, the latter classification scheme is more appropriate because it more easily identifies biochemical targets that are qualitatively different from normal cells.

Some proto-oncogenes are activated to an oncogenic phenotype by the increased or deregulated expression of a normal gene. Proteins encoded by these oncogenes are quantitatively, but not qualitatively, different from the normal protein. Such oncogene products are generally not suitable targets for new antineoplastic drugs due to the decreased basis for tumor selectivity.

Other proto-oncogenes are activated to cellular oncogenes by qualitative mechanisms that produce specific somatic mutations. The proteins encoded by these oncogenes have different structures and biochemical properties from the proteins encoded by the normal genes. These proteins represent biochemical targets that are qualitatively different from those in normal cells and are, therefore, definite targets for anticancer drugs that are selectively toxic to tumor cells.

The present invention provides novel compounds of Formula II

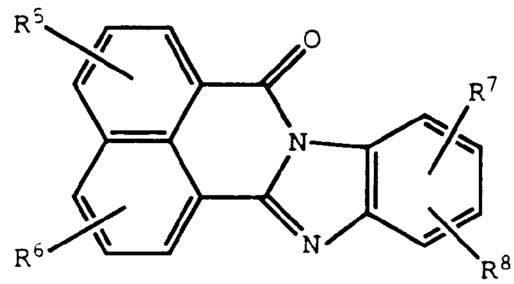

II

wherein:

$R^5$ and $R^6$ are independently selected from the group consisting of hydrogen, carboxy, amide, substituted amide, heterocyclic amines, nitro, halo, hydroxy, hydroxy($C_1$-$C_3$ alkyl), $C_1$-$C_3$ alkoxy, -O-C(O)-$R^f$, -C(O)-$NR^gR^h$ and -$NR^iR^j$, wherein $R^f$, $R^g$, $R^h$, $R^i$, and $R^j$ are independently selected from the group consisting of hydrogen, and $C_1$-$C_6$ alkyl, said alkyl being either branched or straight; or together form an alkylimidazole or benzyl group; and

$R^7$ and $R^8$ are independently selected from the group consisting of hydrogen, halo, amino, nitro, dimethylamino, methylamino, trifluoromethyl, and $C_1$-$C_3$ alkyl; or together form a $C_4$-$C_7$ aryl;

provided that at least one of $R^5$ and $R^6$ is not hydrogen and that at least one of $R^7$ and $R^8$ is not hydrogen; and pharmaceutically acceptable salts or solvates thereof. Such compounds are especially preferred in the treatment of susceptible neoplasms in mammals.

The series of 7H-benzimidazo[2,1-a]benz[*de*]isoquinolines compounds reported herein are useful in the treatment of various cancers, especially those cancers which arise from the activation of oncogenes. Many such isoquinolines are known in the art. Certain of these compounds are known to possess qualities suitable for their use as dyes and pigments and have been used as such. More recently, several derivatives have been described as being useful as epoxy hardeners and as thickening agents for grease. In addition, compounds of the general structural type as those of this invention have also been shown to be useful as immune regulants. J. Campbell, et al., U.S. Patent 4,670,442, issued June 2, 1987.

As used herein, the term "halo" refers to fluoro, chloro, bromo and iodo. As used herein the "$C_1$-$C_6$ alkyl" refers to branched or straight chains of 1 to 6 carbon atoms or cyclic rings of 1 to 6 carbon atoms. As used herein, the term "aryl" refers to carbocyclic groups such as phenyl.

Certain substituted 7H-benzimidazo[2,1-*a*]benz[*de*]isoquinoline-7-ones are named and numbered according to the Ring Index, The American Chemical Society, number 5818, as follows:

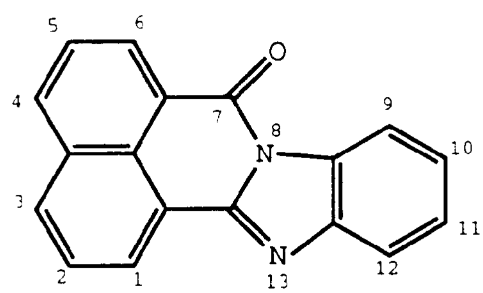

III

and processes of their preparation are taught in the literature.

Okazaki and co-workers describe the preparation of 10- and 11-substituted compounds as represented by Formula II wherein $R^1$ and $R^2$ are hydrogen and one of either $R^3$ or $R^4$ is methyl, methoxy, or chloro. Okazaki, et al., Journal of the Society of Organic and Synthetic Chemistry, Japan, 13:80, 175, 228 and 413 (1955). In a similar manner the synthesis of similar compounds wherein one of either $R^3$ or $R^4$ is chloro, methyl, amino, or nitro has been described. Arient and Marhan, Collection of Czechoslovakian Chemistry Communications, 28:1292 (1963).

Each of the above two references utilizes the same two general processes of preparation. The first process employs the reaction between a substituted 1,8-naphthalic acid anhydride of Formula IV and appropriately

substituted o-phenylenediamines of Formula V

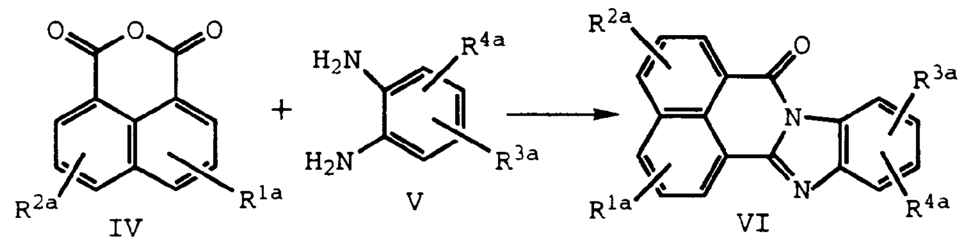

wherein $R^{1a}$, $R^{2a}$, $R^{3a}$ and $R^{4a}$ are either equivalent to $R^1$, $R^2$, $R^3$ and $R^4$, respectively, or can be converted to these moieties using standard techniques.

The substituted phenylenediamines are either prepared and isolated or formed in situ from the appropriate o-nitroaniline precursor. The in situ preparation may be done prior to the introduction of the anhydride or in the presence of the anhydride.

Generally a slight molar excess of the naphthalic anhydride is reacted with the substituted o-phenylenediamine, optionally in the presence of acid-binding agents. The reaction is usually performed in water optionally acidified with catalytic amounts of acid, or in a solvent such as acetic acid, ethanol, N,N-dimethylformamide or N-methylpyrrolidone.

The reaction is complete after several hours at elevated temperature, preferably at about 80°C to about 150°C.

The second process produces an intermediate N-substituted-naphthalimide of Formula VIII from the reaction of a substituted naphthalic anhydride of Formula IV with the appropriately substituted o-nitroaniline of Formula VII

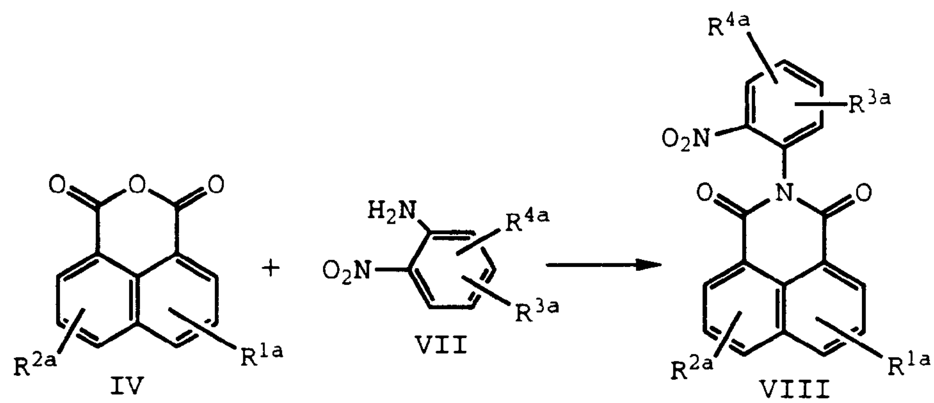

wherein $R^{1a}$, $R^{2a}$, $R^{3a}$ and $R^{4a}$ are either equivalent to $R^1$, $R^2$, $R^3$ and $R^4$, respectively, or can be converted to these moieties using standard techniques.

The resulting compounds of Formula VIII can be converted to compounds of Formula I by chemical reduction and condensation. Heating for several hours to effect cyclization of the imidazole ring is generally required only in those cases with kinetically particularly disadvantageous conditions. The reaction conditions employed by this second process are very similar to those of the first process.

Both processes were employed by Papenfuhs and co-workers who described the reaction of o-phenylenediamines and o-nitroanilines with 3,6-dihydroxynaphthalic acid anhydride to prepare 2,5-dihydroxy congeners of the compounds of Formula III. T. Papenfuhs, et al., U.S. Patent 4,097,450, issued June 27, 1978.

It has been recognized that the second process utilizing o-nitroanilines is superior to the first process when the corresponding o-phenylenediamine reaction can and does result in two isomers. For example, Okazaki, et al., demonstrated a 42:58 ratio of 10- to 11-chloro isomers when 3,4-diaminochlorobenzene was reacted with naphthalic anhydride. Conversely, pure isomers could be obtained when the appropriately substituted o-nitroanilines were first reacted with the anhydride which was followed by chemical reduction and condensation. Okazaki, et al., supra at 413.

Campbell and Lavagnino describe a process for preparing 10- or 11-trifluoromethyl-7H-benzimidazo[2,1-*a*]benz[*de*]isoquinolin-7-one which comprises first reacting naphthalimide with a 4- or 5-trifluoromethyl-substituted o-nitrohalobenzene in an inert solvent in the presence of an alkali metal carbonate or hydroxide to

form the intermediate N-substituted naphthalimide. J.B. Campbell and E.R. Lavagnino, U.S. Patent 4,413,126, issued November 1, 1983. The intermediate is then reduced using means such as catalytic hydrogenation, or chemical reducing agents such as zinc or iron dust in the presence of an acid, to produce the compounds of interest. Id.

Generally, the naphthalimide is allowed to react in the presence of 1.0-1.5 molar equivalents of base and 1-2 molar equivalents of the o-nitrohalobenzene, although other ratios may be used. The reaction between the naphthalimide and the trifluoromethyl-substituted o-nitrohalobenzene is generally performed at temperatures from about 50°C to about 200°C, with the range of about 140°C to about 160°C being most preferred.

Suitable solvents are those which are inert to the reaction process and are appropriate for the temperature desired, such as diglyme, N,N-dimethylformamide, dioxane, and the like. The solvents should be essentially free from water to minimize any undesirable interaction with the o-nitrohalobenzene reagents.

Bases which are capable of forming the naphthalimide salt are employed; preferred are alkali metal carbonates and hydroxides such as potassium carbonate and sodium hydroxide. Small amounts of alkali metal halides, such as potassium fluoride or potassium iodide, serve to catalyze the reaction and it is, therefore, desirable to have such compounds present in the reaction mixture to facilitate the reaction process.

An especially preferred process for preparing the products described in U.S. Patent 4,413,126, discussed supra involves first reacting a substituted naphthalimide with potassium hydroxide in ethanol under nitrogen to produce the potassium naphthalimide of Formula IX

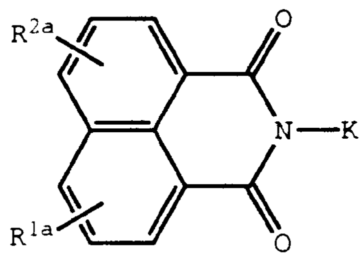

IX

The reactants are usually combined at a ratio of 1.5 - 2.5 moles of potassium hydroxide per one mole of the substituted naphthalimide. The potassium naphthalimide is then reacted with a substituted o-nitrofluorobenzene to form the intermediate N-substituted naphthalimide which is then reduced as described supra. Unlike the process described in U.S. Patent 4,413,126, the reaction of the potassium naphthalimide and the o-nitrofluorobenzene is performed using very mild heat or at room temperature.

Naphthalic anhydride and naphthalimide are commercially available. The o-nitrohalobenzene, o-nitroaniline, and o-phenylenediamine compounds which are required as reactants are either commercially available or can be prepared by the known methods of amination, halogenation, nitration, or reduction of suitable aromatic precursors. The required benzotrifluoride reactants can be prepared by the fluorination of the corresponding benzoic acids with sulfur tetrafluoride.

The compounds of the instant invention are usually prepared as a mixture of regioisomers which can then be separated using conventional techniques, if desired.

The present invention includes methods employing the pharmaceutically acceptable salts of the Formula I compounds as well as the pharmaceutically acceptable salts of the Formula II compounds. The pharmaceutically acceptable acid addition salts of this invention include salts derived from inorganic acids such as hydrochloric acid, nitric acid, phosphoric acid, sulfuric acid, hydrobromic acid, hydroiodic acid, and the like, as well as salts derived from strong organic acids such as aliphatic and aromatic sulfonic acids. Such pharmaceutically acceptable salts thus include sulfate, nitrate, chloride, bromide, iodide, benzenesulfonate, toluenesulfonate, chlorobenzenesulfonate, xylenesulfonate, methanesulfonate, propanesulfonate, naphthalene-1-sulfonate, naphthalene-2-sulfonate, methyl p-toluenesulfonate, and the like salts.

Some of the Formula I and II compounds can react with basic materials such as alkali-metal or alkaline-earth-metal hydroxides, carbonates, and bicarbonates including, without limitation, sodium hydroxide, sodium carbonate, potassium hydroxide, calcium hydroxide, lithium hydroxide, etc. to form pharmaceutically acceptable salts such as the corresponding sodium, potassium, lithium, or calcium salt. Organic bases can also be used, including primary, secondary, and tertiary alkyl amines such as methylamine, triethylamine, and the like.

The terms and abbreviations used in the instant examples have their normal meanings unless otherwise designated. For example, "°C" refers to degrees Celsius; "N" refers to normal or normality; "mmol" refers to

millimole; "g" refers to gram; mL" means milliliter; "µl" refers to microliter; "M" refers to molar or molarity; and "m.s." refers to mass spectrometry.

The following examples further illustrate the preparation of the compounds of the present invention.

Example 1

Preparation of 5-(dimethylamino)-1H,3H-naphtho[1,8-*cd*]pyran-1,3-dione.

3-Nitro-1,8-naphthalene dicarboxylic acid anhydride (0.1 mole, 24.3 g) and 25 g of formaldehyde (0.246 mole) were dissolved in a mixture of 100 mL of tetrahydrofuran and 300 mL of ethyl alcohol. To this mixture was added 5 g of 5% palladium on activated carbon and the mixture was pressurized with 60 p.s.i. of hydrogen in a Parr Hydrogenation apparatus. The reaction mixture was then shaken at 60°C for 2 hours at which time the theoretical amount of hydrogen was taken up. After cooling to room temperature the solid that had separated along with the mixture was refluxed in N,N-dimethylformamide until the title product was in solution. The catalyst was collected on a filter. The title product separated from the cooled filtrate in 55% yield. MP 228-229°C, m.s. 241 ($M^+$)

| Analysis for: $C_{14}H_{11}NO_3$ | | | |
|---|---|---|---|
| | C | H | N |
| Calculated: | 69.70 | 4.60 | 5.81 |
| Found: | 69.68 | 4.73 | 5.59 |

Example 2

Preparation of the regioisomeric compounds: 2-(dimethylamino)-10-nitro-7H-benzimidazo[2,1-*a*]benz[*de*]isoquinolin-7-one; 2-(dimethylamino)-11-nitro-7H-benzimidazo[2,1-*a*]benz[*de*]isoquinolin-7-one; 5-(dimethylamino)-10-nitro-7H-benzimidazo[2,1-*a*]benz[*de*]isoquinolin-7-one; and 5-(dimethylamino)-11-nitro-7H-benzimidazo[2,1-*a*]benz[*de*]isoquinolin-7-one

A mixture of 5-(dimethylamino)-1H,3H-naphtho[1,8-*cd*]pyran-1,3-dione (2.41 g, 10.0 mmol) and 15.3 g (0.1 mole) of 4-nitro-1,2-phenylenediamine was refluxed in 250 mL of N,N-dimethylformamide for 24 hours. The prescription mixture was cooled and the solid that separated was collected and washed with methanol to provide a 65% yield of the four title regioisomers. m.s. 358 ($M^+$).

| Analysis for: $C_{20}H_{14}N_4O_3$ | | | |
|---|---|---|---|
| | C | H | N |
| Calculated: | 67.03 | 3.94 | 15.63 |
| Found: | 66.83 | 4.04 | 15.43 |

Example 3

Preparation of the regioisomeric compounds: 10-amino-2-(dimethylamino)-7H-benzimidazo[2,1-*a*]benz[*de*]isoquinolin-7-one; 11-amino-2-(dimethylamino)-7H-benzimidazo[2,1-*a*]benz[*de*]isoquinolin-7-one; 10-amino-5-(dimethylamino)-7H-benzimidazo[2,1-*a*]benz[*de*]isoquinolin-7-one; and 11-amino-5-(dimethylamino)-7H-benzimidazo[2,1-*a*]benz[*de*]isoquinolin- 7-one.

A Parr Hydrogenation flask containing 9 grams (25.1 mmol) of a mixture of the compound products of Example 2 in 140 mL of tetrahydrofuran, together with 1 g of 5% palladium on activated carbon was shaken at room temperature overnight at an initial pressure of 60 p.s.i. It was then heated at 40°C for 1 hour. After cooling to room temperature the solid and catalyst that separated were collected and refluxed in N,N-dimethylformamide until the solid went into solution and the catalyst could be removed by filtration. The filtrate was then cooled. The red solid that separated from the cooled filtrate was collected and recrystallized from N,N-dimethylformamide to provide a 33% yield of the mixture of 4 regioisomers. m.s. 328 ($M^+$).

| Analysis for: $C_{20}H_{16}N_4O$ | | | |
|---|---|---|---|
| | C | H | N |
| Calculated: | 73.15 | 4.91 | 17.06 |
| Found: | 72.80 | 5.06 | 16.70 |

Example 4

Preparation of the regioisomeric compounds: 2-nitro-10-(trifluoromethyl)-7H-benzimidazo[2,1-*a*]benz[*de*]isoquinolin-7-one; 2-nitro-11-(trifluoromethyl)-7H-benzimidazo[2,1-*a*]benz[*de*]isoquinolin-7-one; 5-nitro-10-(trifluoromethyl)-7H-benzimidazo[2,1-*a*]benz[*de*]isoquinolin-7-one; and 5-nitro-11-(trifluoromethyl)-7H-benzimidazo[2,1-*a*]benz[*de*]isoquinolin-7-one.

4-amino-3-nitrobenzotrifluoride (0.15 mole, 30.9 g) was hydrogenated to the diamine in methanol under 60 p.s.i. using Raney nickel catalyst in a Parr Hydrogenation apparatus. The catalyst was removed by filtration. The filtrate and 0.15 mole (36.5 g) of 3-nitro-1,8-naphthalene dicarboxylic acid anhydride were heated for 48 hours at 180°C. The solid that separated was recrystallized from tetrahydrofuran to yield a mixture of the four title regioisomers. 41% yield. m.s. 383 ($M^+$).

| Analysis for: $C_{19}H_8F_3N_3O_3$ | | | | |
|---|---|---|---|---|
| | C | H | N | F |
| Calculated: | 59.54 | 2.10 | 10.96 | 14.87 |
| Found: | 59.79 | 2.02 | 11.00 | 15.00 |

Example 5

Preparation of the regioisomeric compounds: 5-amino-10-trifluoromethyl-7H-benzimidazo[2,1-*a*]benz[*de*]isoquinolin-7-one; 5-amino-11-trifluoromethyl-7H-benzimidazo[2,1-*a*]benz[*de*]isoquinolin-7-one; 2-amino-10-trifluoromethyl-7H-benzimidazo[2,1-*a*]benz[*de*]isoquinolin-7-one; and 2-amino-11-trifluoromethyl-7H-benzimidazo[2,1-*a*]benz[*de*]isoquinolin-7-one.

The 4 regioisomers of Example 4 (42.1 g, 0.11 mole) were hydrogenated in 950 mL of tetrahydrofuran at ambient temperature for 4 hours at 60 p.s.i. using 8 g of 5% palladium on activated carbon as catalyst . After removal of the catalyst and solvent, the title products were recrystallized from a 3:1 solution of tetrahydrofuran: methanol. The yield was 76%. m.s. 353 ($M^+$).

| Analysis for: $C_{19}H_{10}F_3N_3O$ | | | | |
|---|---|---|---|---|
| | C | H | N | F |
| Calculated: | 64.59 | 2.85 | 11.89 | 16.13 |
| Found: | 64.77 | 2.61 | 11.68 | 15.89 |

Example 6

Separation of the compounds of Example 4.

The mixture of product regioisomers of Example 4 was separated into the individual compounds by careful preparative HPLC chromatography using a gradient. The instrument used was a Waters Associates Prep LC System 500. The separated isomers were identified by high resolution NMR techniques.

Example 7

Preparation of 5-amino-10-(trifluoromethyl)-7H-benzimidazo[2,1-*a*]benz[*de*]isoquinolin-7-one.

5-Nitro-10-(trifluoromethyl)-7H-benzimidazo[2,1-*a*]benz[*de*]isoquinolin-7-one (4.15 g, 0.0108 mole) was dissolved in 95 mL of tetrahydrofuran. One gram of 5% palladium on activated carbon was added and the mixture hydrogenated at 60 p.s.i. at ambient temperature for 2 hours. More tetrahydrofuran was added as some solid product had separated out of the reaction. The mixture was boiled and filtered hot to remove the catalyst. Upon cooling, a 53% yield of the title product was collected. m.s. 353 ($M^+$). Melting point 282°C-283°C.

| Analysis for: $C_{19}H_{10}F_3N_3O$ | | | |
|---|---|---|---|
| | C | H | N |
| Calculated: | 64.59 | 2.85 | 11.89 |
| Found: | 64.54 | 2.72 | 11.87 |

Example 8

Preparation of 5-amino-11-(trifluoromethyl)-7H-benzimidazo[2,1-*a*]benz[*de*]isoquinolin-7-one.

5-Nitro-11-(trifluoromethyl)-7H-benzimidazo[2,1-*a*]benz[*de*]isoquinolin-7-one (7.66 g, 0.02 mole) was dissolved in 90 of tetrahydrofuran. One gram of 5% palladium on activated carbon was added and the mixture hydrogenated at 60 p.s.i. at ambient temperature for 1 1/2 hours. During this reduction the temperature exothermed to 45°C. The mixture was filtered and the product and catalyst were collected and heated in N,N-dimethylformamide. The reaction mixture was then filtered hot to remove the catalyst. The N,N-dimethylformamide filtrate was chilled overnight and the title product was collected in a 52% yield. Melting point 279°C-280°C. m.s. 353 ($M^+$).

| Analysis for: $C_{19}H_{10}F_3N_3O$ | | | |
|---|---|---|---|
| | C | H | N |
| Calculated: | 64.59 | 2.85 | 11.89 |
| Found: | 64.68 | 3.08 | 11.87 |

Example 9

Preparation of 2-amino-10-(trifluoromethyl)-7H-benzimidazo[2,1-*a*]benz[*de*]isoquinolin-7-one.

2-Nitro-10-(trifluoromethyl)-7H-benzimidazo[2,1-*a*]benz[*de*]isoquinolin-7-one (3.5 g, 9.1 mmol) was dissolved in 95 mL of tetrahydrofuran. One gram of 5% palladium on activated carbon was added and the mixture hydrogenated at 60 p.s.i. for 1 hour at ambient temperature. The temperature exothermed to 35°C during this time. The catalyst was removed by filtration and the filtrate allowed to stand until crystals separated. The crystals were collected providing an 88% yield of the title product. Melting point 279°C-281°C, m.s. 353 ($M^+$).

| Analysis for: $C_{19}H_{10}F_3N_3O$ | | | |
|---|---|---|---|
| | C | H | N |
| Calculated: | 64.59 | 2.85 | 11.89 |
| Found: | 64.49 | 3.16 | 11.63 |

Example 10

Preparation of 2-amino-11-(trifluoromethyl)-7H-benzimidazo[2,1-*a*]benz[*de*]isoquinolin-7-one.

2-Nitro-11-(trifluoromethyl)-7H-benzimidazo[2,1-*a*]benz[*de*]isoquinolin-7-one (4 g, 10.4 mmol) was dissolved in 95 mL of tetrahydrofuran. One gram of 5% palladium on activated carbon was added and the mixture hydrogenated at 60 p.s.i. for 1 hour at ambient temperature. The catalyst was removed by filtration and the filtrate allowed to stand overnight. The next day a 65% yield of the title product was collected as a solid. Melting point 264°C-265°C.

| Analysis for: $C_{19}H_{10}F_3N_3O$ | | | |
|---|---|---|---|
| | C | H | N |
| Calculated: | 64.59 | 2.85 | 11.89 |
| Found: | 64.37 | 3.03 | 11.76 |

Example 11

Preparation of the regioisomeric compounds: 3-nitro-10-chloro-7H-benzimidazo[2,1-*a*]benz[*de*]isoquinolin-7-one; 3-nitro-11-chloro-7H-benzimidazo[2,1-*a*]benz[*de*]isoquinolin-7-one; 4-nitro-10-chloro-7H-benzimidazo[2,1-*a*]benz[*de*]isoquinolin-7-one; and 4-nitro-11-chloro-7H-benzimidazo[2,1-*a*]benz[*de*]isoquinolin-7-one.

4-Nitronaphthalic anhydride (24.3 g, 0.1 mole) was slurried in 600 mL of acetic acid. The 4-nitronaphthalic anhydride was prepared in substantial accordance with known procedures. See, e.g., L. Jones, et al., Canadian Journal of Chemistry, 48:3132 (1970). The slurry was heated to reflux and 14.3 grams (0.1 mole) of 4-chloro-o-phenylenediamine, dissolved in 200 mL of hot acetic acid, was added. After five minutes the solution darkened and a precipitate began to form. The solution was refluxed for 6 hours and was then allowed to cool overnight. The tan precipitate (29.8 g) was collected and dried. m.s. 349 ($M^+$).

| Analysis for: $C_{18}H_8ClN_3O_3$ | | | |
|---|---|---|---|
| | C | H | N |
| Calculated: | 61.82 | 2.31 | 12.02 |
| Found: | 62.04 | 2.56 | 12.08 |

Example 12

Preparation of the regioisomeric compounds: 2-[(2-methylpropyl)amino]-10-chloro-7H-benzimidazo[2,1-*a*]benz[*de*]isoquinolin-7-one; 2-[(2-methylpropyl)amino]-11-chloro-7H-benzimidazo[2,1-*a*]benz[*de*]isoquinolin-7-one; 5-[(2-methylpropyl)amino]-10-chloro-7H-benzimidazo[2,1-*a*]benz[*de*]isoquinolin-7-one; and 5-[(2-methylpropyl)amino]-11-chloro-7H-benzimidazo[2,1-*a*]benz[*de*]isoquinolin-7-one.

3-Nitro-1,8-naphthalic anhydride (24.3 g, 0.1 mole) was slurried in 500 mL of tetrahydrofuran. The 3-nitronaphthalic anhydride was prepared in substantial accordance with known procedures. See, e.g., L. Jones, et al., Canadian Journal of Chemistry, 48:3132 (1970). To this slurry was added 7.2 g (0.1 mole) of *t*-butyraldehyde. Five grams of 5% palladium on activated carbon was added and the mixture hydrogenated at 60 p.s.i. overnight at ambient temperature. The catalyst and solvent were removed. The 3-(2-methylpropyl)amino-naphthalic anhydride was recovered and then washed with acetone.

A slurry of 3-(2-methylpropyl)amino-naphthalic anhydride (13.5 g, 50 mmol) in 100 mL of tetrahydrofuran was heated to reflux and 7.1 grams (0.05 mole) of 4-chloro-o-phenylenediamine was added. After five minutes the solution darkened and a precipitate began to form. The solution was refluxed for 21 hours and was then allowed to cool. The reaction mixture was then poured into cold water. The mixture of title compounds was then recrystallized from acetone. The mixture was collected and dried. m.s. 375 ($M^+$);

| Analysis for: $C_{22}H_{18}ClN_3O$ | | | |
|---|---|---|---|
| | C | H | N |
| Calculated: | 70.30 | 4.83 | 11.18 |
| Found: | 70.02 | 4.76 | 10.95 |

Example 13

Preparation of the regioisomeric compounds: 3-(pentylamino)-9-nitro-7H-benzimidazo[2,1-*a*]benz[*de*]isoquinolin-7-one; 3-(pentylamino)-12-nitro-7H-benzimidazo[2,1-*a*]benz[*de*]isoquinolin-7-one; 4-(pentylamino)-12-nitro-7H-benzimidazo[2,1-*a*]benz[*de*]isoquinolin-7-one; and 4-(pentylamino)-9-nitro-7H-benzimidazo[2,1-*a*]benz[*de*]isoquinolin-7-one.

In 300 mL of absolute ethanol were added 25 g (0.16 mole) of 3-nitro-1,2-phenylenediamine and 38 g (0.16 mole) of 4-chloro-1,8-naphthalic anhydride. This mixture was heated to 180°C and maintained at this temperature for about 16 hours. The solid precipitate, a mixture of 3-(chloro)-9-nitro-7H-benzimidazo[2,1-*a*]benz[*de*]isoquinolin-7-one; 3-(chloro)-12-nitro-7H-benzimidazo[2,1-*a*]benz[*de*]isoquinolin-7-one; 4-(chloro)-12-nitro-7H-benzimidazo[2,1-*a*]benz[*de*]isoquinolin-7-one; and 4-(chloro)-9-nitro-7H-benzimidazo[2,1-*a*]benz[*de*]isoquinolin-7-one was collected and recrystallized from N,N-dimethylformamide.

To a solution of 150 of N,N-dimethylformamide and 25 mL of triethylamine were added 18.1 g (0.05 mole) of the regioisomeric mixture prepared above and 8.7 g (0.1 mole) of 1-aminopentane. This slurry was heated to 180°C and was maintained at this temperature for about 12 hours, after which time the reaction mixture was poured into a bath of cold water. The solid was collected and recrystallized from acetone, giving a 47% yield of the mixture of the title compounds.
m.s. 400 ($M^+$);

| Analysis for: $C_{23}H_{20}N_4O_3$ | | | |
|---|---|---|---|
| | C | H | N |
| Calculated: | 68.99 | 5.03 | 13.99 |
| Found: | 68.76 | 5.14 | 14.29 |

Example 14

Preparation of the regioisomeric compounds: 2,12-diamino-10-chloro-7H-benzimidazo[2,1-*a*]benz[*de*]isoquinolin-7-one; 2,9-diamino-11-chloro-7H-benzimidazo[2,1-*a*]benz[*de*]isoquinolin-7-one; 5,9-diamino-11-chloro-7H-benzimidazo[2,1-*a*]benz[*de*]isoquinolin-7-one; and 2,12-diamino-10-chloro-7H-benzimidazo[2,1-*a*]benz[*de*]isoquinolin-7-one.

To a solution of 400 mL of absolute ethanol were added 3-nitro-1,8-naphthalic anhydride (24.3 g, 0.1 mole) and 3-nitro-5-chloro-1,2-phenylenediamine (18.8 g, 0.1 mole). This solution was heated to 180°C and was kept at this temperature for about 16 hours. The solid precipitate, containing a mixture of the regioisomers 2,12-dinitro-10-chloro-7H-benzimidazo[2,1-*a*]benz[*de*]isoquinolin-7-one; 2,9-dinitro-11-chloro-7H-benzimidazo[2,1-*a*]benz[*de*]isoquinolin-7-one; 5,9-dinitro-11-chloro-7H-benzimidazo[2,1-*a*]benz[*de*]isoquinolin-7-one; and 2,12-dinitro-10-chloro-7H-benzimidazo[2,1-*a*]benz[*de*]isoquinolin-7-one was collected.

The crude mixture collected supra was then suspended in 500 mL of tetrahydrofuran. To this slurry was added 10 g of palladium on activated carbon. This reaction mixture was incubated under hydrogen purge (60 p.s.i.) at room temperature for about two hours. The slurry was then heated to 60°C and was incubated at this temperature for about 2 hours. The catalyst was then removed and the solid precipitate, containing the mixture of the title compounds, was recrystallized from acetone. m.s. 334 ($M^+$);

| Analysis for: $C_{18}H_{11}ClN_4O$ | | | |
|---|---|---|---|
| | C | H | N |
| Calculated: | 64.58 | 3.31 | 16.74 |
| Found: | 64.37 | 3.55 | 14.95 |

Table 1 describes the substitution pattern of many of the compounds of Formula I which were prepared and used for subsequent testing. Column 1 provides the number to which the compound is referred in subsequent tables. Columns 2-5 describe the substitutions at positions 2, 5, 10 and 11 on the multiple ring structure. Column 6 depicts other substitutions on the multiple ring structure. The absence of a substituent group listed at a particular ring position indicates that hydrogen is bound at that position. The ring positions are numbered as indicated below:

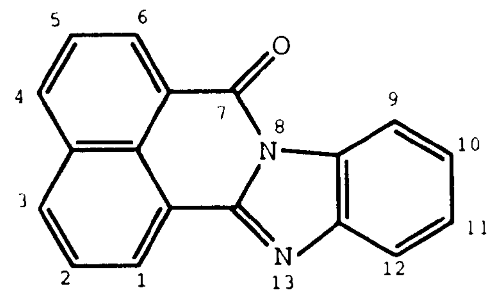

| Compound No. | Ring Position | | | |
|---|---|---|---|---|
| | 2 | 5 | 10 | 11 | Other |
| 1 | $-NO_2$ | | $-CF_3$ | | |
| 2 | $-NO_2$ | | | $-CF_3$ | |
| 3 | | $-NO_2$ | | $-CF_3$ | |
| 4 | | $-NO_2$ | $-CF_3$ | | |
| 5 | | $-NH_2$ | $-CF_3$ | | |
| 6 | | $-NH_2$ | | $-CF_3$ | |
| 7 | $-NH_2$ | | $-CF_3$ | | |
| 8 | $-NH_2$ | | | $-CF_3$ | |
| 9 | $-NH_2$ | $-NH_2$ | $-CF_3$ | | |

| Compound No. | Ring Position | | | | |
|---|---|---|---|---|---|
| | 2 | 5 | 10 | 11 | Other |
| 10 | | | $-F$ | | $3-N(CH_3)_2$ |
| 11 | | | | $-F$ | $3-N(CH_3)_2$ |
| 12 | | | $-F$ | | $4-N(CH_3)_2$ |
| 13 | | | | $-F$ | $4-N(CH_3)_2$ |
| 14 | | | $-Cl$ | $-F$ | $3-Cl$ |
| 15 | | | $-F$ | $-Cl$ | $3-Cl$ |
| 16 | | | $-Cl$ | $-F$ | $4-Cl$ |
| 17 | | | $-F$ | $-Cl$ | $4-Cl$ |
| 18 | $-N(CH_3)_2$ | | $-NO_2$ | | |
| 19 | $-N(CH_3)_2$ | | | $-NO_2$ | |
| 20 | | $-N(CH_3)_2$ | $-NO_2$ | | |
| 21 | | $-N(CH_3)_2$ | | $-NO_2$ | |
| 22 | $-N(CH_3)_2$ | | | | $9-CH_3$ |
| 23 | $-N(CH_3)_2$ | | | | $12-CH_3$ |

| Compound No. | Ring Position | | | | |
|---|---|---|---|---|---|
| | 2 | 5 | 10 | 11 | Other |
| 24 | | | | | 6-$N(CH_3)_2$ 9-$CH_3$ |
| 25 | | | | | 6-$N(CH_3)_2$ 12-$CH_3$ |
| 26 | $-N(CH_3)_2$ | | -Cl | | |
| 27 | $-N(CH_3)_2$ | | | -Cl | |
| 28 | | $-N(CH_3)_2$ | -Cl | | |
| 29 | | $-N(CH_3)_2$ | | -Cl | |
| 30 | | | -Cl | | 3-$NHCH_3$ |
| 31 | | | | -Cl | 3-$NHCH_3$ |
| 32 | | | -Cl | | 4-$NHCH_3$ |
| 33 | | | | -Cl | 4-$NHCH_3$ |
| 34 | $-N(CH_3)_2$ | | $-NH_2$ | | |
| 35 | $-N(CH_3)_2$ | | | $-NH_2$ | |
| 36 | | $-N(CH_3)_2$ | $-NH_2$ | | |

| Compound No. | Ring Position | | | | |
|---|---|---|---|---|---|
| | 2 | 5 | 10 | 11 | Other |
| 37 | | $-N(CH_3)_2$ | | $-NH_2$ | |
| 38 | | | -Cl | | 3-Cl<br>12-$NO_2$ |
| 39 | | | | -Cl | 3-Cl<br>9-$NO_2$ |
| 40 | | | -Cl | | 4-Cl<br>12-$NO_2$ |
| 41 | | | | -Cl | 4-Cl<br>9-$NO_2$ |
| 42 | $-NO_2$ | $-NO_2$ | $-CF_3$ | | |
| 43 | $-NO_2$ | $-NO_2$ | | $-CF_3$ | |
| 44 | | | $-CH_2OH$ | | 3-Cl |
| 45 | | | | $-CH_2OH$ | 3-Cl |
| 46 | | | $-CH_2OH$ | | 4-Cl |
| 47 | | | | $-CH_2OH$ | 4-Cl |
| 48 | | | $-NH_2$ | | 3-Cl |
| 49 | | | | $-NH_2$ | 3-Cl |

| Compound No. | Ring Position | | | | |
|---|---|---|---|---|---|
| | 2 | 5 | 10 | 11 | Other |
| 50 | | | $-NH_2$ | | 4-Cl |
| 51 | | | | $-NH_2$ | 4-Cl |
| 52 | | | | | 3-Cl<br>9-OH |
| 53 | | | | | 3-Cl<br>12-OH |
| 54 | | | | | 4-Cl<br>9-OH |
| 55 | | | | | 4-Cl<br>12-OH |
| 56 | $-NO_2$ | | | | 9-$CF_3$ |
| 57 | $-NO_2$ | | | | 12-$CF_3$ |
| 58 | | $-NO_2$ | | | 9-$CF_3$ |
| 59 | | $-NO_2$ | | | 12-$CF_3$ |
| 60 | | | $-CF_3$ | | 3-$NH_2$ |
| 61 | | | | $-CF_3$ | 3-$NH_2$ |
| 62 | | | $-CF_3$ | | 4-$NH_2$ |

| Compound No. | Ring Position | | | | |
|---|---|---|---|---|---|
| | 2 | 5 | 10 | 11 | Other |
| 63 | | | | $-CF_3$ | $4-NH_2$ |
| 64 | $-NH_2$ | $-NH_2$ | | $-CF_3$ | |
| 65 | $-NO_2$ | | | $-CF_3$ | $9-NH_2$ |
| 66 | | $-NO_2$ | | $-CF_3$ | $9-NH_2$ |
| 67 | $-NO_2$ | | $-CF_3$ | | $12-NH_2$ |
| 68 | | $-NO_2$ | $-CF_3$ | | $12-NH_2$ |
| 69 | | | $-Cl$ | | $3-NHCH_2CH_2CH_3$ |
| 70 | | | | $-Cl$ | $3-NHCH_2CH_2CH_3$ |
| 71 | | | $-Cl$ | | $4-NHCH_2CH_2CH_3$ |
| 72 | | | | $-Cl$ | $4-NHCH_2CH_2CH_3$ |
| 73 | | | $-Cl$ | | $3-NH_2$ |
| 74 | | | | $-Cl$ | $3-NH_2$ |
| 75 | | | $-Cl$ | | $4-NH_2$ |
| 76 | | | | $-Cl$ | $4-NH_2$ |
| 77 | | | $-NH_2$ | | $3-N(CH_3)_2$ |
| 78 | | | | $-NH_2$ | $3-N(CH_3)_2$ |
| 79 | | | $-NH_2$ | | $4-N(CH_3)_2$ |

| Compound No. | Ring Position | | | | |
|---|---|---|---|---|---|
| | 2 | 5 | 10 | 11 | Other |
| 80 | | | | $-NH_2$ | $4-N(CH_3)_2$ |
| 81 | $-NHCH_2CH(CH_3)_2$ | | -Cl | | |
| 82 | $-NHCH_2CH(CH_3)_2$ | | | -Cl | |
| 83 | | $-NHCH_2CH(CH_3)_2$ | -Cl | | |
| 84 | | $-NHCH_2CH(CH_3)_2$ | | -Cl | |
| 85 | | | $-NH_2$ | | $3-NHCH_2CH_2CH_3$ |
| 86 | | | $-NH_2$ | | $4-NHCH_2CH_2CH_3$ |
| 87 | | | | $-NH_2$ | $4-NHCH_2CH_2CH_3$ |
| 88 | | | | | $9-NO_2$<br>$3-NH(C_5H_{11})$ |
| 89 | | | | | $12-NO_2$<br>$3-NH(C_5H_{11})$ |
| 90 | | | | | $9-NO_2$<br>$4-NH(C_5H_{11})$ |
| 91 | | | | | $12-NO_2$<br>$3-NH(C_5H_{11})$ |
| 92 | | | $-NH(C_5H_{11})$ | | 3-Cl |

| Compound No. | Ring Position | | | | |
|---|---|---|---|---|---|
| | 2 | 5 | 10 | 11 | Other |
| 93 | | | | $-NH(C_5H_{11})$ | 3-Cl |
| 94 | | | $-NH(C_5H_{11})$ | | 4-Cl |
| 95 | | | | $-NH(C_5H_{11})$ | 4-Cl |
| 96 | | | -Cl | | $3-N(CH_3)_2$ |
| 97 | | | -Cl | | $4-N(CH_3)_2$ |
| 98 | | | | -Cl | $3-N(CH_3)_2$ |
| 99 | | | | -Cl | $4-N(CH_3)_2$ |
| 100 | $-NHCH_2CH_2CH_3$ | | $-CF_3$ | | |
| 101 | $-NHCH_2CH_2CH_3$ | | | $-CF_3$ | |
| 102 | | $-NHCH_2CH_2CH_3$ | $-CF_3$ | | |
| 103 | | $-NHCH_2CH_2CH_3$ | | $-CF_3$ | |
| 104 | | $-NH_2$ | | $-NH_2$ | |
| 105 | | $-NH_2$ | $-NH_2$ | | |
| 106 | $-NH_2$ | | | $-NH_2$ | |
| 107 | $-NH_2$ | | $-NH_2$ | | |

| Compound No. | Ring Position | | | | |
|---|---|---|---|---|---|
| | 2 | 5 | 10 | 11 | Other |
| 108 | | | | $-NH_2$ | $3-NHCH_2CH_2CH_3$ |
| 109 | | | -Cl | | $3,\ 4-(CH)_2C_4H_8ONH$ |
| 110 | | | | -Cl | $3,\ 4-(CH)_2C_4H_8ONH$ |
| 111 | | | -Cl | | $5,\ 6-(CH)_2C_4H_8ONH$ |
| 112 | | | | -Cl | $5,\ 6-(CH)_2C_4H_8ONH$ |
| 113 | | | $-CF_3$ | | $3-C_2HNCHNCHCH$ |
| 114 | | | | $-CF_3$ | $3-C_2HNCHNCHCH$ |
| 115 | | | $-CF_3$ | | $4-C_2HNCHNCHCH$ |
| 116 | | | | $-CF_3$ | $4-C_2HNCHNCHCH$ |
| 117 | $NHCOCH_3$ | | $-CF_3$ | | |
| 118 | | $NHCOCH_3$ | $-CF_3$ | | |
| 119 | $NHCOCH_3$ | | | $-CF_3$ | |
| 120 | | $NHCOCH_3$ | | $-CF_3$ | |
| 121 | | | | $-C(OH)(CF_3)_2$ | |
| 122 | | | $-C(OH)(CF_3)_2$ | | |

| Compound No. | Ring Position | | | | |
|---|---|---|---|---|---|
| | 2 | 5 | 10 | 11 | Other |
| 123 | | | $-SO_2H$ | | |
| 124 | | | $-SO_2H$ | | |
| 125 | | | -SCN | | |
| 126 | | | | -SCN | |
| 127 | | | $-SCH_3$ | | |
| 128 | | | | $-SCH_3$ | |

The present invention also provides a method of treating a susceptible neoplasm in a mammal which comprises administering to a mammal in need of said treatment an effective amount of a compound of Formula I:

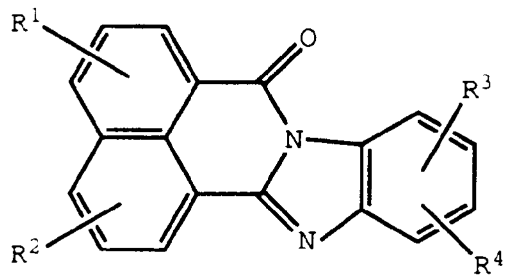

I

wherein:

$R^1$ and $R^2$ are independently selected from the group consisting of hydrogen, carboxy, amide, substituted amide, heterocyclic amines, nitro, halo, hydroxy, $C_1$-$C_3$ alkyl, hydroxy($C_1$-$C_3$ alkyl), $C_1$-$C_3$ alkoxy, -O-C(O)-$R^a$, -C(O)-$NR^bR^c$ and -$NR^dR^e$, wherein $R^a$, $R^b$, $R^c$, $R^d$, and $R^e$ are independently selected from the group consisting of hydrogen, and $C_1$-$C_6$ alkyl; or together form an alkylimidazole or benzyl group; and

$R^3$ and $R^4$ are independently selected from the group consisting of hydrogen, halo, amino, nitro, dimethylamino, methylamino, trifluoromethyl, and $C_1$-$C_3$ alkyl; or together form a $C_4$-$C_7$ aryl;

provided that at least one of $R^1$ and $R^2$ is not hydrogen; and further provided that at least one of $R^3$ and $R^4$ is not hydrogen; or a pharmaceutically acceptable salt or solvate thereof.

Preferred methods of treatment employ compounds of Formula I in which $R^1$ and $R^2$ are independently selected from the group consisting of hydrogen, dimethylamino, methylamino, amino, and nitro; and $R^3$ and $R^4$ are independently selected from the group consisting of hydrogen, nitro, chloro, fluoro, trifluoromethyl and amino.

In particular, the present compounds are useful in treating solid tumors including carcinomas such as ovarian, non-small cell lung, gastric, pancreatic, prostate, renal cell, breast, colorectal, small cell lung, melanoma, and head and neck; and sarcomas such as Kaposi's sarcoma and rhabdomyosarcoma. These compounds have been shown to be especially effective against those carcinomas and sarcomas which arise from activation of the ras oncogene.

Many of the compounds of Formula I have been shown to be effective antineoplastic agents using both in vitro and in vivo model test systems.

For some of the in vitro and in vivo test systems, normal rat kidney epithelial (NRKE) cells [J. DeLarco and G.J. Todaro, Journal of Cell Physiology, 94:335 (1978)] were employed. Stock cultures of these cell lines were obtained from the American Type Culture Collection and are readily available. Both cell lines were grown in antibiotic-free Dulbecco's high glucose Modified Eagle's Medium supplemented with 10% fetal bovine serum.

The cell number of the NRKE cell line was expanded by 5 passages and stored in liquid nitrogen. These cryopreserved cells were used for the methods described below. All cell lines were grown using standard cell culture methods. R.I. Freshney, Culture of Animal Cells: A Manual of Basic Technique, p. 127 (2d ed. 1987). All cell culture manipulations were done under gold fluorescent light to prevent damage by photo-oxidation.

Initially, NRKE cells transformed by the $ras^K$ and $ras^H$ were used to determine the selective cytotoxic action of compounds on ras-transformed epithelial cells. Only transformants with a minimal deviation phenotype were used. Transformants with this phenotype have a low oncogene copy number and in vitro growth characteristics the same as nontransformed NRKE cells. These criteria were chosen so that compounds were evaluated for antitumor activity, rather than anti-growth activity based on the rate of cell division.

The procedure used to create the ras oncogene-transformed NRKE cells for this study employed plasmids pUCEJ6.6 [ATCC 41038, described in Molecular and Cellular Biology, 5:1400-1407 (1985)], containing a transforming human $ras^H$ gene, pKSma [ATCC 41048, described in Biochemical and Biophysical Research Communications, 108:1631-1637 (1982)], containing a v-$ras^K$ gene and the RSV neo gene. These plasmids were obtained from the American Type Culture Collection, Rockville, Maryland and are freely available to the public. NRKE cells were co-transfected with an oncogene plus the RSV neo gene using standard calcium phosphate coprecipitation and neomycin resistance selection methods. See, e.g., L.G. Davis, et al., Basic Methods in Molecular Biology, p. 285 (1986). The neo gene provides resistance to antibiotics such as neomycin and its analogs.

Approximately 14 days after transfection and selection using the antibiotic G418 (geneticin), a neomycin analog, colonies were isolated by the ring cloning procedure. Freshney, supra. The clones were stored in liquid nitrogen after expansion of their cell number by 4 passages in medium supplemented with G418.

Approximately 50 clones were evaluated by several criteria for their use in the in vitro cytotoxicity and in vivo antitumor tests described below. The clones KNRK [ATCC CRL 1569, described in J. Gen. Virol. 13: 245-52 (1971)], transformed by v-$ras^K$ gene, and EJ/1.2-NRKE, transformed by the human $ras^H$ gene, were employed in the in vitro cytotoxicity and in vivo antitumor testing. These clones have 3 to 4 oncogene copies that are stably integrated. The in vitro doubling time of these cells is 24 hours, which is the same as NRKE cells.

Adenocarcinomas were produced in female CD1 *nu/nu* nude mice approximately 3 days after the subcutaneous implantation of 1 x $10^6$ cells. These tumors grow with a doubling time of 24 hours. In contrast, no tumors were produced in nude mice up to one year after the subcutaneous implantation of 108 parent NRKE cells.

In vitro cytotoxicity assay

The compounds of Formula I were screened for antitumor activity with an oncogene-based Differential Cytotoxicity Assay. With this assay the cytotoxic action of compounds against oncogene transformed cells relative to their action against the nontransformed parent epithelial cells was determined. For the evaluation of compounds, the Lewis lung carcinoma was used as a murine reference tumor. This tumor is commonly used in screens for new oncolytics.

The Differential Cytotoxicity Assay can identify compounds that interact directly with oncogene proteins, but more importantly it is designed to find compounds that interfere with the biochemical pathways activated or driven by oncogenes. The inhibitory action in both cases may be specific for a transformed cell.

Briefly, the procedure used for the Differential Cytotoxicity Assay is as follows. Cell suspensions were prepared by trypsin dissociation and the cells were seeded into each well of 12-well culture dishes. Groups of triplicate wells were divided into media control, drug vehicle-control and drug treatment groups. One day after seeding, the media was replaced with media containing vehicle or drugs and the cultures incubated for an additional 5 days. After exposure to the vehicles or drugs, the cultures were washed, fixed and stained with a modification of Mallory's stain. K.C. Richardson, et al., Stain Technology, 35:313 (1960).

Colony number and colony area were determined with an image analyzer such as that produced by Artek System Corp., Farmington, NY (Artek model 982 image analyzer). The cytocidal action of the compounds was determined from the colony number using standard techniques. T.T. Puck and P.I. Marcus, Journal of Experimental Medicine, 103:653 (1956). Colony areas were normalized for colony number and were used to determine if a compound had cytostatic activity.

Table 2, infra, depicts the results of one such series of in vitro cytotoxicity studies. Column 1 provides the compound numbers as detailed in Table 1, supra. In these initial screens, usually a mixture of isomers was used, even though the individual isomers may first be separated using known methods. Columns 2, 3 and 4 depict the relative cytotoxicity of the different benzimidazobenzisoquinolones. The cytotoxicity was measured by counting the number of colonies in the negative control plates relative to the number of colonies in the treated plates for the three cell lines listed, normal rat kidney epithelial cells (NRKE), $ras^K$-transformed rat kidney epithelial cells (KNRK), and the well studied Lewis Lung carcinoma (3LL). Unless otherwise indicated, all of the treated samples were exposed to a 33$\mu$M concentration of the benzimidazobenzisoquinolone.

Table 2

In Vitro Evaluation of the Differential Cytotoxicity of Benzimidazobenzisoquinolones

| | Relative Cytotoxicity (Treated/Control) | | |
|---|---|---|---|
| Compound No.† | NRKE | KNRK | 3LL |
| 1-4 | 1.00 | 0.01 | 0.00 |
| 38-41 | 0.21 | 0.05 | 1.29 |
| 42-43 | 1.25 | 0.00 | 0.00 |
| 44-47 | 0.61 | 0.39 | 0.00 |
| 48-51 | 0.17 | 0.01 | 0.01 |
| 48-51* | 0.81 | 0.00 | 0.80 |
| 65-68 | 0.69 | 0.54 | 0.60 |
| 52-55 | 0.17 | 0.02 | 0.12 |
| 56-59 | 1.16 | 0.45 | 0.49 |
| 60-63 | 0.87 | 0.00 | 0.31 |
| 9,64‡ | 0.94 | 0.00 | 0.94 |
| 9,64‡* | 1.25 | 0.35 | 1.05 |
| 73-76 | 0.52 | 0.00 | 0.00 |
| 10-13 | 1.19 | 0.80 | 0.64 |
| 69-72 | 0.95 | 0.74 | 0.09 |
| 81-84 | 1.00 | 0.11 | 0.57 |
| 77-80 | 0.39 | 0.09 | 0.01 |
| 85-87 | 0.89 | 0.23 | 0.05 |
| 88-91 | 1.17 | 0.69 | 0.02 |
| 92-95 | 0.54 | 0.09 | 0.03 |
| 96-99 | 1.30 | 1.16 | 0.00 |
| 100-103 | 0.58 | 0.20 | 0.43 |

†Indicates that a mixture of compounds was used (e.g. 56-59 indicates that a mixture of Compound Numbers 56, 57, 58 and 59 was used).

‡Indicates that an equimolar mixture of Compound Numbers 9 and 64 was used.

*Indicates that the test compound was present at a concentration of 3.3 µM.

Only those compounds that demonstrated substantial cytocidal activity in this primary screen were evaluated further in the animal tumor models described below. The concentration for a 50% cell kill ($LC_{50}$) was determined, and a differential index of cytotoxicity was determined by dividing the $LC_{50}$ value for the normal epithelial cells by the $LC_{50}$ value for the tumor cells. The differential index value is used to determine the

amount of selective toxicity a compound has for the tumor cells. One example of such a study is shown in Table 3.

Table 3

Differential Toxicity of the Individual Isomers of Compounds 34-37 and the Mixture of these Isomers Against NRKE And KNRK

| Compound No(s). | LC50 (μM) NRKE | KNRK | Differential Index* |
|---|---|---|---|
| 34-37† | 18.3 | 0.26 | 70.4 |
| 34 | >33.3 | 0.065 | >512.3 |
| 35 | >33.3 | 0.35 | >95.1 |
| 36 | 12.5 | 0.11 | 113.6 |
| 37 | >33.3 | 1.59 | >20.9 |

*Differential Index = $LC_{50}$ NRKE/$LC_{50}$ KNRK
†Indicates a mixture of the compounds depicted by the range.

In vivo antitumor assay.

Female CD1 nu/nu nude mice were obtained from Charles Rivers, Inc. The animal procedures used were those standard in the field. See e.g., R. L. Merriman, et al., Cancer Research, 49:4509 (1989). Briefly, the animals were housed in plastic shoebox-type cages covered with a microisolator top. All animal feed, water, bedding and cages were sterilized. Food and water were provided ad libitum.

All animal manipulations were done with sterile procedures in a 100% exhaust, vertical laminar flow, HEPA filtered hood. To maintain consistent tumor growth, only mice between 4 and 6 weeks of age were used. Merriman, et al., supra.

Transformed cells, obtained by ras oncogene transfection of NRKE cells (KNRK), were grown as xenograft tumors in female CD1 nu/nu mice. Transformed cells grown in vitro to the seventh passage were used to establish the xenografts.

An animal tumor model was used to further evaluate the antitumor activity of compounds that have cytocidal activity in the Differential Cytotoxicity Assay. Tumors were initiated by the subcutaneous implantation of 1 x $10^6$ cells approximately 1 cm from the first mammary gland. After implantation the mice were randomized and divided into treatment groups of between 7 and 10 mice per group. Starting one day after tumor implantation, the mice were dosed with drug daily for 10 days, usually intraperitoneally.

On day 11, tumor mass was determined essentially as described by J.F. Worzalla, et al., Investigational New Drugs, 8:241-251 (1990) using digital electronic calipers interfaced to a microcomputer. Tumor weights were calculated from these measurements using the following formula:

$$\text{Tumor weight (mg)} = \frac{\text{tumor length (mm)} \times [\text{tumor width (mm)}]^2}{2}$$

Alternatively, tumor weights can be calculated using the procedures taught by Tomayko and co-workers. M.M. Tomayko, and C.P. Reynolds, Cancer Chemotherapy and Pharmacology, 24:148 (1989)

At least one control group of an equal number of mice was treated with the same volume of the carrier only. The percent inhibition of tumor growth was calculated from the ratio of the tumor mass for the drug treated animals relative to the tumor mass for the control group of animals. All animals were weighed at the beginning and end of treatment to determine if inhibition of tumor growth was due to weight loss.

Table 4 provides one example of the type of testing done in vivo using the KNRK xenograft model. A mixture of the compounds of Examples 34-37 was administered by one of the three routes detailed in column 2: intraperitoneally (IP); orally (PO); or subcutaneously (SC). Column 3 describes the dosage level of the compound in milligrams per kilogram of body weight. Column 4 describes the percent inhibition of tumor growth. Column 5 tallies the number of mice which died during the course of the experiment relative to the total number of animals in that group.

Table 4

| In Vivo antitumor Activity of Compounds 34-37 Using Various Routes of Drug Administration | | | |
|---|---|---|---|
| Route | Dosage | Percent Inhibition | Toxic/Total |
| IP | 150 | 95 ± 6 | 0/9 |
| IP | 300 | 98 ± 6 | 2/10 |
| PO | 150 | 67 ± 19 | 0/10 |
| PO | 300 | 89 ± 10 | 0/10 |
| SC | 150 | 89 ± 9 | 0/10 |
| SC | 300 | 95 ± 6 | 0/10 |

Table 5 depicts additional in vivo studies using the KNRK xenograft model described supra. In these studies, different compounds were employed to demonstrate the anti-neoplastic activity of the compounds of this invention.

Table 5

Antitumor Activity Of Benzimidazobenzisoquinolones in the KNRK Xenograft Model

| Example No(s).[†] | Dosage | Percent Inhibition | Toxic/Total |
|---|---|---|---|
| 34-37 | 150 | 98 ± 7 | 0/9 |
| | 300 | 89 ± 10 | 0/9 |
| 5 | 50 | 47 ± 13 | 0/9 |
| | 100 | 65 ± 9 | 0/9 |
| 6 | 100 | 22 ± 25 | 0/9 |
| | 200 | 47 ± 23 | 0/9 |
| 7 | 100 | 55 ± 20 | 0/9 |
| | 200 | 75 ± 15 | 0/9 |
| {154049} | 25 | 79 ± 16 | 0/9 |
| 8 | 50 | 81 ± 11 | 0/9 |
| {213878} | 30 | 75 ± 10 | 1/9 |
| 9 | 100 | --- | 9/9 |
| {228525-6- | 50 | 91 ± 14 | 0/9 |
| 7-8} | 100 | 91 ± 23 | 5/9 |
| 10-13 | | | |
| {253651-54} | 150 | 89 ± 4 | 1/9 |
| 14-17 | 300 | 93 ± 4 | 1/9 |
| {258150-1- | 150 | 81 ± 9 | 0/9 |
| 2-3} | 300 | 88 ± 6 | 2/9 |
| 22-25 | | | |
| {264238-41} | 150 | 96 ± 4 | 3/9 |
| 26-29 | 300 | 94 ± 3 | 7/9 |
| {264394-5- | 150 | 92 ± 4 | 0/7 |
| 6-7} | 300 | 93 ± 4 | 1/7 |
| 30-33 | | | |

[†] a range of compounds (e.g. 34-37) indicates a mixture of the isomers depicted by that range.

The results of several experiments in mice and <u>in vitro</u> demonstrated that many of the compounds of Formula I are effective antineoplastic agents which serve to suppress tumor growth.

The present invention also provides pharmaceutical formulations comprising an effective amount for treating susceptible neoplasms of the compounds of Formulas I and II in combination with a suitable pharmaceutical carrier, diluent, or excipient. These formulations are useful in the treatment of mammals suffering from susceptible neoplasms. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The pharmaceutical compositions of the present invention contain, as the active ingredient, the compounds of Formulas I and II associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range. For example, dosages per day normally fall within the range of about 0.5 to about 600 mg/kg of body weight. In the treatment of adult humans, the range of about 1 to about 50 mg/kg, in single or divided dose, is preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way.

Formulation Example 1

Hard gelatin capsules containing the following ingredients are prepared:

| Ingredient | Quantity (mg/capsule) |
|---|---|
| 10-Amino-2-(dimethylamino)-7H-benzimidazo[2,1-*a*]benz[*de*]isoquinolin-7-one | 250.0 |
| Starch | 305.0 |
| Magnesium stearate | 5.0 |

The above ingredients are mixed and filled into hard gelatin capsules in 560 mg quantities.

Formulation Example 2

A tablet formula is prepared using the ingredients below:

| Ingredient | Quantity (mg/tablet) |
|---|---|
| 11-Amino-2-(methylamino)-7H-benzimidazo[2,1-*a*]benz[*de*]isoquinolin-7-one | 250.0 |
| Cellulose, microcrystalline | 400.0 |
| Colloidal silicon dioxide | 10.0 |
| Stearic acid | 5.0 |

The components are blended and compressed to form tablets, each weighing 665 mg.

Formulation Example 3

A dry powder inhaler formulation is prepared containing the following components:

| Ingredient | Weight % |
|---|---|
| 10-Amino-5-(dimethylamino)-7H-benzimidazo[2,1-*a*]benz[*de*]isoquinolin-7-one | 5 |
| Lactose | 95 |

The active mixture is mixed with the lactose and the mixture is added to a dry powder inhaling appliance.

Formulation Example 4

Tablets, each containing 60 mg of active ingredient, are prepared as follows:

| Ingredient | Quantity (mg/tablet) |
|---|---|
| 11-Amino-4-(dimethylamino)-7H-benzimidazo[2,1-*a*]benz[*de*]isoquinolin-7-one | 60.0 gm |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 150 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

Formulation Example 5

Capsules, each containing 80 mg of medicament are made as follows:

| Ingredient | Quantity (mg/capsule) |
|---|---|
| 2-Amino-10-(chloro)-7H-benzimidazo[2,1-*a*]benz[*de*]isoquinolin-7-one | 80.0 mg |
| Starch | 109.0 mg |
| Magnesium stearate | 1.0 mg |
| Total | 190.0 mg |

The active ingredient, cellulose, starch, and magnesium stearate are blended, passed through a No. 20 mesh U.S. sieve, and filled into hard gelatin capsules in 190 mg quantities.

Formulation Example 6

Suppositories, each containing 225 mg of active ingredient are made as follows:

| Ingredient | Amount |
|---|---|
| 2-Nitro-11-(trifluoromethyl)-7H-benzimidazo[2,1-*a*]benz[*de*]isoquinolin-7-one | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 mL dose are made as follows:

| Ingredient | Amount |
|---|---|
| 3-Methylamino-10-(fluoro)-7H-benzimidazo[2,1-*a*]benz[*de*]isoquinolin-7-one | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor | q.v. |
| Color | q.v. |
| Purified water to | 5.0 mL |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 150 mg of medicament, are made as follows:

| Ingredient | Quantity (mg/capsule) |
|---|---|
| 5-Dimethylamino-11-(trifluoromethyl)-7H-benzimidazo[2,1-*a*]benz[*de*]isoquinolin-7-one | 150.0 mg |
| Starch | 407.0 mg |
| Magnesium stearate | 3.0 mg |
| Total | 560.0 mg |

The active ingredient, cellulose, starch, and magnesium stearate are blended, passed through a No. 20 mesh U.S. sieve, and filled into hard gelatin capsules in 560 mg quantities.

Formulation Example 9

An intravenous formulation may be prepared as follows:

| Ingredient | Quantity (mg/capsule) |
|---|---|
| 11-Amino-5-(dimethylamino-7H-benzimidazo[2,1-*a*]benz[*de*]isoquinolin-7-one | 250.0 mg |
| Isotonic saline | 1000 mL |

The solution of the above ingredients is administered intravenously at a rate of 1 mL per minute to a subject in need of treatment.

**Claims**

1. A compound of the formula

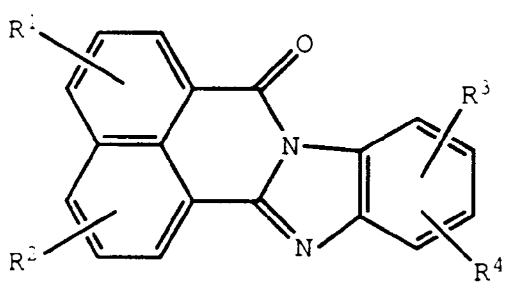

wherein:

R¹ and R² are independently selected from the group consisting of hydrogen, carboxy, amide, substituted amide, heterocyclic amine, nitro, halo, hydroxy, $C_1$-$C_3$ alkyl, hydroxy($C_1$-$C_3$ alkyl), $C_1$-$C_3$ alkoxy, -O-C(O)-$R^a$, -C(O)-$NR^bR^c$ and -$NR^dR^e$, wherein $R^a$, $R^b$, $R^c$, $R^d$, and $R^e$ are independently selected from the group consisting of hydrogen, and $C_1$-$C_6$ alkyl, said alkyl being either branched or straight or cyclic, or together form an alkylimidazole or benzyl group; and

$R^3$ and $R^4$ are independently selected from the group consisting of hydrogen, halo, amino, nitro, dimethylamino, methylamino, trifluoromethyl, thiocyanate, thioalkyl, sulfonyl, and $C_1$-$C_3$ alkyl; or together form a $C_4$-$C_7$ aryl;

provided that at least one of $R^1$ and $R^2$ is not hydrogen and that at least one of $R^3$ and $R^4$ is not hydrogen; or a pharmaceutically acceptable salt or solvate thereof, for use in treating neoplasms.

2. A compound as claimed in Claim 1 for treatring a neoplasm caused by the activation of an oncogene.

3. A compound as claimed in Claim 2 wherein the oncogene is the ras oncogene.

4. A compound as claimed in Claim 1 wherein at least one of $R^3$ and $R^4$ is selected from the group consisting

of $-NH_2$, halo, $C_1$-$C_3$ alkyl, and trifluoromethyl.

5. A compound as claimed in Claim 1 wherein at least one of $R^1$ and $R^2$ is selected from the group consisting of $-NR^dR^e$ and halo.

6. A compound as claimed in Claim 5 selected from the group consisting of 10-amino-2-(dimethylamino)-7H-benzimidazo[2,1-*a*]benz[*de*]isoquinolin-7-one; 11-amino-2-(dimethylamino)-7H-benzimidazo[2,1-*a*]benz[*de*]isoquinolin-7-one; 10-amino-5-(dimethylamino)-7H-benzimidazo[2,1-*a*]benz[*de*]isoquinolin-7-one; and 11-amino-5-(dimethylamino)-7H-benzimidazo[2,1-*a*]benz[*de*]isoquinolin-7-one.

7. A compound of the formula

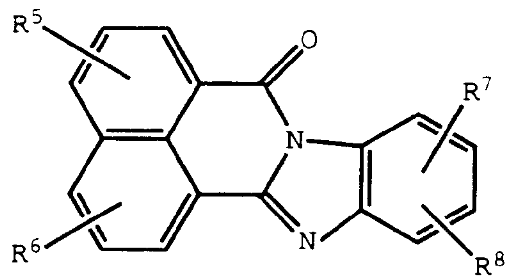

wherein:

$R^5$ and $R^6$ are independently selected from the group consisting of hydrogen, carboxy, amide, substituted amide, heterocyclic amine, nitro, halo, hydroxy, $C_1$-$C_3$ alkyl, hydroxy($C_1$-$C_3$ alkyl), $C_1$-$C_3$ alkoxy, $-O-C(O)-R^f$, $-C(O)-NR^gR^h$ and $-NR^iR^j$, wherein $R^f$, $R^g$, $R^h$, $R^i$, and $R^j$ are independently selected from the group consisting of hydrogen, and $C_1$-$C_6$ alkyl, said alkyl being either branched or straight or cyclic; or together form an alkylimidazole or benzyl group; and

$R^7$ and $R^8$ are independently selected from the group comprising hydrogen, halo, amino, nitro, dimethylamino, methylamino, trifluoromethyl, and $C_1$-$C_3$ alkyl; or together form a $C_4$-$C_7$ aryl;

provided that at least one of $R^5$ and $R^6$ is not hydrogen and that at least one of $R^7$ and $R^8$ is not hydrogen; or a pharmaceutically acceptable salt or solvate thereof.

8. A compound as claimed in Claim 7 wherein at least one of $R^3$ and $R^4$ is selected from the group consisting of $-NH_2$, halo, $C_1$-$C_3$ alkyl and trifluoromethyl.

9. A compound as claimed in Claim 7 wherein at least one of $R^1$ and $R^2$ is selected from the group consisting of $-NR^dR^e$ and halo.

10. A compound as claimed in Claim 9 selected from the group consisting of 10-amino-2-(dimethylamino)-7H-benzimidazo[2,1-*a*]benz[de]isoquinolin-7-one; 11-amino-2-(dimethylamino)-7H-benzimidazo[2,1-*a*]benz[*de*]isoquinolin-7-one; 10-amino-5-(dimethylamino)-7H-benzimidazo[2,1-*a*]benz[*de*]isoquinolin-7-one; and 11-amino-5-(dimethylamino)-7H-benzimidazo[2,1-*a*]benz[*de*]isoquinolin-7-one.

11. A pharmaceutical formulation comprising a compound of the formula

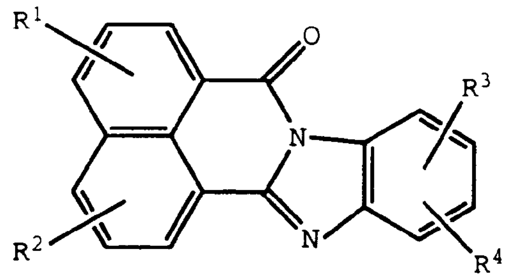

wherein:

$R^1$ and $R^2$ are independently selected from the group consisting of hydrogen, carboxy, amide, substituted amide, heterocyclic amine, nitro, halo, hydroxy, $C_1$-$C_3$ alkyl, hydroxy($C_1$-$C_3$ alkyl), $C_1$-$C_3$ alkoxy,

-O-C(O)-$R^a$, -C(O)-$NR^bR^c$ and $NR^dR^e$, wherein $R^a$, $R^b$, $R^c$, $R^d$, and $R^e$ are independently selected from the group consisting of hydrogen, and $C_1$-$C_6$ alkyl, said alkyl being either branched or straight or cyclic; or together form an alkylimidazole or benzyl group; and

$R^3$ and $R^4$ are independently selected from the group consisting of hydrogen, halo, amino, nitro, dimethylamino, methylamino, trifluoromethyl, and $C_1$-$C_3$ alkyl; or together form a $C_4$-$C_7$ aryl;

provided that at least one of $R^1$ and $R^2$ is not hydrogen and that at least one of $R^3$ and $R^4$ is not hydrogen;

or a pharmaceutically acceptable salt or solvate thereof, as defined in Claims 1 to 5, associated with a suitable pharmaceutical excipient.

European Patent Office

# EUROPEAN SEARCH REPORT

Application Number
EP 93 31 0354
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF THE SOCIETY OF DYERS AND COLOURISTS<br>vol. 85, no. 6, 1969, ENGLAND<br>pages 246 - 251<br>J. ARIENT ET AL 'Imidazole dyes. XX'<br>*see Tables 4 and 5*<br>--- | 7,8 | C07D471/06<br>A61K31/47<br>//(C07D471/06,235:00,221:00) |
| X | CHEMIA ANALITYCZNA<br>vol. 29, no. 2, 1984, WARSAW<br>pages 221 - 230<br>J. JUSIAK ET AL 'Thin layer chromatographic isolation of synthetic products'<br>*see Table 1*<br>--- | 7,8 | |
| X | DE-A-1 962 092 (FARBENFABRIKEN BAYER)<br>16 June 1971<br>*see claim 4 and formulae III and IV, page 3*<br>--- | 7,8 | |
| X | PATENT ABSTRACTS OF JAPAN<br>vol. 13, no. 495 (P-956)9 November 1989<br>& JP-A-11 98 762 ( MITSUBISHI KASEI CORP ) 10 August 1989<br>* abstract *<br>*see page 874,871 of JP-A-1198762*<br>--- | 7,8,9 | **TECHNICAL FIELDS SEARCHED (Int. Cl.5)**<br>C07D<br>A61K |
| X | PATENT ABSTRACTS OF JAPAN<br>vol. 13, no. 502 (P-958)13 November 1989<br>& JP-A-12 01 671 ( KONICA CORP. ) 14 August 1989<br>* abstract *<br>*see pages 712/713 of JP-A-1201671*<br>---<br>-/-- | 7,8 | |

The present search report has been drawn up for all claims

| Place of search | Date of completion of the search | Examiner |
|---|---|---|
| MUNICH | 01 MARCH 1994 | SCRUTON-EVANS I. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number
EP 93 31 0354
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN vol. 13, no. 502 (P-958)13 November 1989 & JP-A-12 01 670 ( KONICA CORP. ) 14 August 1989 * abstract * *see page 696 or JP-A-1201670* --- | 7,8 | |
| X | CHEMICAL ABSTRACTS, vol. 60, no. 6, 1964, Columbus, Ohio, US; abstract no. 6955d, 'Imidazole dyes. XII.' column 6955 ; * abstract * & COLLECTION CZECH. CHEM. COMMUN. vol. 28, 1963, pages 3352 - 3361 J. ARIENT ET AL *see all of the dinitro compounds* --- | 7 | |
| X | CHEMICAL ABSTRACTS, vol. 62, no. 9, 1965, Columbus, Ohio, US; abstract no. 10574d, 'disperse dyes' * abstract * & JP-A-6 427 119 (MITSUBISHI CHEM IND CO LTD.) 27 November 1964 *see compounds of Tables* & JP-A-6 427 120 (MITSUBISHI CHEM IND. CO LTD) 27 November 1964 --- | 7 | TECHNICAL FIELDS SEARCHED (Int. Cl.5) |

-/--

The present search report has been drawn up for all claims

| Place of search | Date of completion of the search | Examiner |
|---|---|---|
| MUNICH | 01 MARCH 1994 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number
EP 93 31 0354
Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 87, 1977, Columbus, Ohio, US; abstract no. 153423m, 'water soluble dyes for wool' page 66 ; * abstract * & JP-A-7 772 726 (MITSUBISHI CHEM IND CO LTD) 17 June 1977 *see compound of formula I wherein R2R3=benzo and R=Br* | 7,8 | |
| X | CHEMICAL ABSTRACTS, vol. 105, 1986, Columbus, Ohio, US; abstract no. 235744w, 'electrophotographic photoreceptor' page 595 ; * abstract * & JP-A-6 167 865 (MITSUBISHI CHEM IND CO LTD) 8 April 1986 **2-hydroxy and 5-hydroxy-7H-benzimidazo[2,1-a]benz(de)isoquinolin-7-one* | 7 | |
| D,X | US-A-4 097 450 (THEODOR PAPENFUHS) 27 June 1978 *see examples 5,8,9,13-17,21,24,27,29,30* | 7,8 | |
| A | CHEMICAL ABSTRACTS, vol. 59, 1963, Columbus, Ohio, US; abstract no. 4070f, 'Imidazole dyes VII.' * abstract * | 1-11 | |
| D,A | & COLLECTION CZECH CHEM COMMUN vol. 28, 1963, pages 1292 - 1300 J.ARIENT ET AL | 1-11 | |

TECHNICAL FIELDS SEARCHED (Int. Cl.5)

-/--

The present search report has been drawn up for all claims

| Place of search | Date of completion of the search | Examiner |
|---|---|---|
| MUNICH | 01 MARCH 1994 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number
EP 93 31 0354
Page 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 57, 1957, Columbus, Ohio, US; abstract no. 2745h, 'acenaphthene series I,II,III,IV' * abstract * | 1-11 | |
| D,A | & J.SOC.ORG.SYNTHET.CHEM.,JAPAN vol. 13, 1955, pages 80 - 4 MITSUO OKAZAKI --- | 1-11 | |
| D,A | US-A-4 670 442 (JACK B. CAMPBELL) 2 June 1987 * the whole document * --- | 1-11 | |
| A | EP-A-0 502 668 (BRITISH TECHNOLOGY GROUP LTD) 9 September 1992 * the whole document * --- | 1-11 | |
| A | EP-A-0 511 792 (ELI LILLY AND COMPANY) 4 November 1992 * the whole document * --- | 1-11 | TECHNICAL FIELDS SEARCHED (Int. Cl.5) |
| A | WO-A-9 203 136 (ABBOTT LABORATORIES) 5 March 1992 * the whole document * ----- | 1-11 | |

The present search report has been drawn up for all claims

| Place of search | Date of completion of the search | Examiner |
|---|---|---|
| MUNICH | 01 MARCH 1994 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)